# FASCICULE DE BREVET EUROPEEN

(11) **EP 1 448 109 B1**
(45) Date de publication et mention de la délivrance du brevet: **24.09.2014**
(21) Numéro de dépôt: 02796885.8
(22) Date de dépôt: 28.11.2002
(51) Int. Cl.: A61B 17/70

(54) **IMPLANT INTERVERTEBRAL A CALE ELASTIQUEMENT DEFORMABLE**
ZWISCHENWIRBELIMPLANTAT MIT ELASTISCHEM, VERFORMBAREM ZWISCHENSTÜCK
INTERVERTEBRAL IMPLANT WITH ELASTICALLY DEFORMABLE WEDGE

(30) Priorité: 30.11.2001 FR 0115494
(43) Date de publication de la demande: 25.08.2004
(73) Titulaire: ZIMMER SPINE, 33000 Bordeaux (FR)
(72) Inventeur: SENEGAS, Jacques, F-33700 Mérignac (FR); LE COUEDIC, Régis, F-78570 ANDRESY (FR)
(74) Mandataire: Venner Shipley LLP
(86) Numéro de dépôt international: PCT/FR2002/004083
(87) Numéro de publication internationale: WO 2003/045262

(56) Documents cités:
- EP-A- 0 322 334
- DE-A- 2 821 678
- FR-A- 2 681 525
- FR-A- 2 704 745
- FR-A- 2 717 675
- FR-A- 2 775 183
- US-A- 5 989 291

## Description

La présente invention concerne un implant intervertébral comportant une cale destinée à s'appliquer entre deux apophyses épineuses de deux vertèbres du rachis.

Des implants intervertébraux bien connus comprennent une cale destinée à être insérée entre les apophyses épineuses qui prolongent la partie postérieure des vertèbres pour limiter et contrôler le déplacement relatif des vertèbres les unes par rapport aux autres. Ces implants sont généralement installés sur le rachis de patients atteints d'une pathologie dégénérative dudit rachis pour lesquels les vertèbres sont susceptibles de se rapprocher les unes des autres et de comprimer, par exemple, les racines nerveuses. Il a été imaginé une première catégorie de cales entièrement rigides et réalisées en une seule pièce présentant deux extrémités opposées susceptibles d'être reliées, respectivement, à deux apophyses épineuses contiguës par des moyens de liaison. Ainsi, lorsque le rachis est étendu, par exemple, le rapprochement de la partie postérieure de deux vertèbres est limité par la cale contre laquelle s'appuient les apophyses épineuses ; et lorsqu'il est courbé vers l'avant les apophyses épineuses sont retenues l'une par rapport à l'autre grâce aux moyens de liaison. Toutefois, ce calage rigide ne reproduit pas fidèlement les conditions physiologiques réelles de limitation du mouvement des vertèbres les unes par rapport aux autres. Pour pallier ce problème il a été imaginé une deuxième catégorie de cales, réalisées dans un matériau élastiquement déformable permettant de reproduire aussi fidèlement que possible les conditions du déplacement relatif contrôlé des vertèbres les unes par rapport aux autres lors des mouvements du rachis. De la sorte, les forces tendant à repousser les vertèbres l'une de l'autre s'accroissent lorsque les vertèbres se déplacent l'une vers l'autre.

Cependant, les matériaux élastiquement déformables utilisés et aptes à être comprimés pour limiter le rapprochement des vertèbres les unes par rapport aux autres se déforment longitudinalement, de façon trop importante par rapport aux déplacements des vertèbres d'un individu normal.

Un problème qui se pose et que vise à résoudre la présente invention est alors de réaliser une cale qui non seulement permette de contrôler le rapprochement progressif des vertèbres les unes par rapport aux autres pour le limiter mais aussi, qui permettent de bloquer les apophyses épineuses des vertèbres lorsqu'elles sont entraînées dans des directions opposées l'une de l'autre. Une telle réalisation est proposée dans le document FR-2717675.

A cet effet, la présente invention propose un implant intervertébral comportant une cale, ladite cale comprenant : deux éléments présentant chacun une première partie adaptée pour être reliée à une apophyse épineuse et une deuxième partie d'appui, opposée à ladite première partie, les deuxièmes parties d'appui étant situées en regard l'une de l'autre ; des moyens élastiquement compressibles disposés entre lesdites deuxièmes parties d'appui, lesdits moyens élastiquement compressibles étant comprimés par lesdites deuxièmes parties d'appui lors de l'entraînement desdits deux éléments l'un vers l'autre ; et, des moyens de liaison distincts des moyens compressibles pour relier lesdits deux éléments entre eux, lesdits moyens de liaison permettant de bloquer lesdits deux éléments en translation l'un par rapport à l'autre lorsque lesdits deux éléments sont entraînés dans des directions opposées l'une de l'autre.

Ainsi, une caractéristique de l'implant intervertébral conforme à l'invention réside dans le mode de liaison entre eux des deux éléments solidaires des apophyses épineuses par des moyens de liaison permettant de bloquer lesdits éléments en translation lorsqu'ils sont éloignés l'un de l'autre, et par des moyens élastiquement compressibles interposés entre les deuxièmes parties d'appui qui maintiennent les éléments espacés l'un de l'autre. De la sorte, la cale se comprime longitudinalement lorsque les apophyses se rapprochent l'une de l'autre, l'effort que les premières parties exercent sur elles pour les éloigner étant proportionnel à la compression des moyens élastiquement compressibles et donc au déplacement relatif des apophyses l'une par rapport à l'autre, et la cale bloque les apophyses lorsqu'elles tendent à s'éloigner l'une de l'autre après que les moyens élastiquement compressibles ont retrouvé leur état de repos dans ledit implant.

On comprend que les moyens élastiquement compressibles ne sont mis en oeuvre que dans la phase de compression, et qu'ils exercent leur fonction uniquement dans cette phase. Dans la phase d'extension, ils ne sont nullement sollicités, seuls le sont les moyens de liaison dont la fonction est de bloquer de manière rigide le déplacement des apophyses épineuses dans des directions opposées l'une de l'autre. On peut ainsi régler séparément le rapprochement des apophyses et leur écartement.

Selon un mode de mise en oeuvre de l'invention particulièrement avantageux, lesdits moyens de liaison comprennent au moins un chemin de passage traversant chacun desdits éléments et débouchant sensiblement de chaque côté de ladite deuxième partie d'appui. De la sorte, lesdits éléments sont susceptibles d'être maintenus, leur deuxième partie en regard l'une de l'autre, de façon parfaitement symétrique de chaque côté de ladite partie d'appui. De façon préférentielle, lesdits moyens de liaison comportent un lien continu formant boucle, ledit lien présentant deux premières portions opposées traversant respectivement lesdits deux éléments en regard. Ainsi, le lien est rendu solidaire des deux éléments de façon à les bloquer l'un par rapport à l'autre lorsqu'ils sont éloignés l'un de l'autre, le lien étant tendu. Avantageusement, le lien emprunte le chemin de passage traversant lesdits éléments.

Selon un mode préférentiel de réalisation de l'invention chacun desdits éléments présente au moins une première portion et une deuxième portion situées sensiblement de chaque côté de ladite deuxième partie d'appui, la première portion et la deuxième portion de l'un desdits éléments étant susceptibles de s'appliquer respectivement contre la deuxième portion et la première portion de l'autre élément lorsque lesdits deux éléments sont entraînés l'un vers l'autre de façon à les bloquer en translation l'un par rapport à l'autre, les deuxièmes parties d'appui étant susceptibles de comprimer lesdits moyens élastiquement compressibles. Ainsi, selon cette caractéristique, la compressibilité de la cale est limitée par les premières et secondes portions des deux éléments. La cale est alors susceptible de se déformer entre une première position de repos, où respectivement, les premières et deuxièmes portions desdits éléments sont maintenues espacées les unes des autres et dans laquelle les moyens élastiquement compressibles, légèrement comprimés entre les deuxièmes parties d'appui, maintiennent les moyens de liaison en extension, et une seconde position d'arrêt où les premières et secondes portions desdits éléments sont respectivement en contact et dans laquelle les deuxièmes parties d'appui compriment lesdits moyens élastiquement compressibles.

Bien évidemment, la compressibilité des moyens élastiquement compressibles d'une part et l'espace entre les premières et deuxièmes portions desdits éléments dans ladite position de repos d'autre part, sont ajustés de façon que l'effort qu'exercent les moyens élastiquement compressibles sur les deuxièmes parties d'appui en regard lorsqu'ils sont comprimés, soit important lorsque les premières et deuxièmes portions entrent en contact. Ainsi, la cale joue pleinement son rôle amortissant sans que les éléments puissent entrer en contact de façon violente.

Selon une caractéristique avantageuse, les éléments de la cale présentent une paroi antérieure susceptible d'être appliquée au regard dudit rachis et une paroi postérieure opposée à ladite paroi antérieure, et ladite première portion et ladite deuxième portion desdits éléments s'étendent sensiblement parallèlement entre elles, de ladite paroi antérieure vers ladite paroi postérieure. De la sorte, comme on l'expliquera plus en détail dans la description détaillée d'un mode de réalisation de l'invention, les premières et secondes portions qui permettent non seulement de bloquer les éléments l'un contre l'autre, permettent également de maintenir plus aisément les moyens élastiquement compressibles.

Préférentiellement, lesdites deuxièmes parties desdits deux éléments situées en regard l'une de l'autre et lesdites première et deuxième portions respectives définissent sensiblement un volume débouchant dans les parois antérieures et les parois postérieures desdits deux éléments, lesdits moyens élastiquement compressibles s'étendant dans ledit volume. Ainsi, lesdits moyens élastiquement compressibles sont susceptibles d'être introduit entre les éléments de la cale sans inconvénients, lorsque ceux-ci sont réunis ensemble par lesdits moyens de liaison, depuis les parois postérieures ou les parois antérieures.

De façon avantageuse, ladite deuxième partie d'appui desdits éléments définit un plan moyen, ladite première portion desdits éléments prolongeant ladite deuxième partie d'appui sensiblement parallèlement audit plan moyen et ladite deuxième portion desdits éléments prolongeant ladite deuxième partie sensiblement perpendiculairement audit plan moyen. Ainsi, ladite première portion de l'un des éléments est susceptible de recevoir en contact ladite deuxième portion de l'autre élément qui fait saillie par rapport au plan moyen de la deuxième partie d'appui de l'autre élément tandis que sa deuxième portion qui fait saillie par rapport au plan moyen de sa deuxième partie d'appui est, elle, susceptible de faire contact avec ladite première portion de l'autre élément. De la sorte, ledit volume est sensiblement défini par les deuxièmes parties d'appui opposées en regard et par lesdites secondes portions situées également en regard l'une de l'autre.

Selon un mode de mise en oeuvre particulièrement avantageux, ledit chemin de passage traversant lesdits éléments débouche dans lesdites première et deuxième portions et se prolonge sensiblement perpendiculairement auxdits plans moyens desdites deuxièmes parties d'appui. De la sorte, les moyens de liaison des deux éléments dont les deuxièmes parties d'appui sont disposées en regard l'une de l'autre, exercent une force sensiblement perpendiculaire audit plan moyen de chaque côté des deuxièmes parties d'appui, ce qui permet une répartition équilibrée des forces de traction sur les éléments de la cale. Cette disposition est d'autant plus avantageuse lorsque préférentiellement, le lien continu formant boucle est constitué d'une bande continue en matériau flexible.

Lorsque le lien est flexible, il est susceptible de se plier aisément dès que les éléments de la cale sont entraînés l'un vers l'autre par les apophyses épineuses.

Préférentiellement, lesdits éléments de la cale sont réalisés dans un matériau rigide de façon à ne pas se déformer sous les contraintes des apophyses épineuses et à comprimer les moyens élastiquement compressibles. Avantageusement, lesdits moyens élastiquement compressibles sont formés d'une seule pièce en élastomère. Les élastomères constituent une famille de composés élastiquement compressibles présentant un faible seuil d'hystérésis, ce qui est particulièrement avantageux pour la cale.

Selon un mode de réalisation particulièrement avantageux, chacune desdites premières parties desdits éléments comporte en outre des moyens d'accrochage pour relier lesdites premières parties auxdites apophyses épineuses desdites vertèbres. Comme on l'expliquera dans la description détaillée qui suivra, ces moyens d'accrochage sont généralement flexibles. Toutefois, des moyens rigides sont susceptibles d'être utilisés.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique en perspective d'un implant intervertébral comportant une cale conforme à l'invention ;
- la Figure 2, est une vue schématique en écorché de la cale représentée sur la Figure 1 ;
- la Figure 3 est une vue schématique de dessus de l'implant représenté sur la Figure 1 selon III ;
- la Figure 4 est une vue schématique en coupe verticale de la cale représentée sur la Figure 3, selon IV ; et,
- la Figure 5 est une vue schématique de côté de la cale telle que représentée sur la Figure 4, selon V.

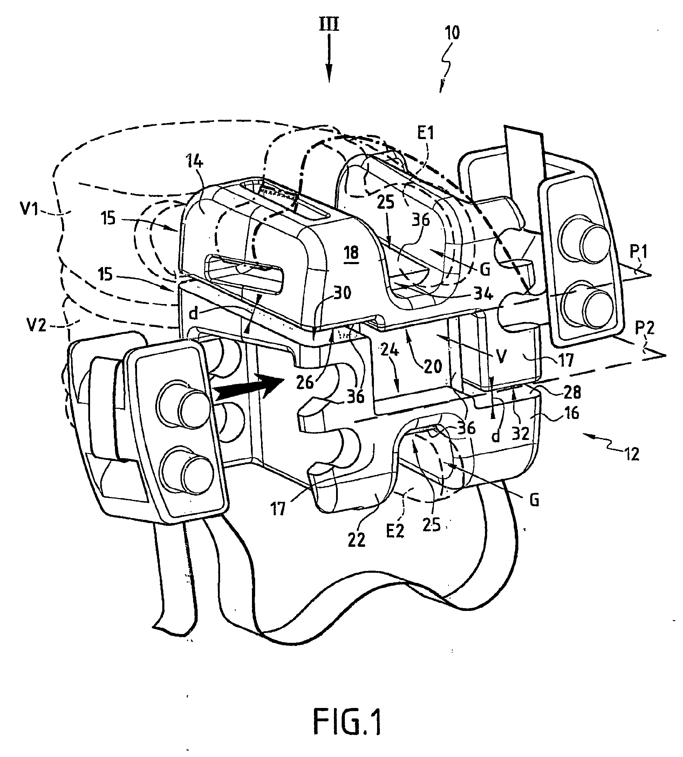

La Figure 1 illustre un implant intervertébral 10, comportant une cale 12, insérée entre deux apophyses épineuses E1 et E2 respectivement de deux vertèbres V1 et V2 et représentées en traits interrompus fins. La cale 12 comporte deux éléments 14, 16, dont les parois antérieures 15 sont disposées au regard des vertèbre V1 et V2 du rachis et les parois postérieures 17 dans la direction opposée. Le premier élément 14 présente une première partie 18 reliée à l'apophyse épineuse E1 et une deuxième partie d'appui 20 opposée à la première partie 18 et définissant un plan moyen P1. Le second élément 16 présente également une première partie 22 qui est reliée à l'apophyse épineuse E2 et une deuxième partie d'appui 24 opposée, définissant un plan moyen P2 et située au regard de la deuxième partie d'appui 20 du premier élément 14. Les premières parties 18 et 22 de chaque élément 14 et 16 sont formées d'une gorge G entre les parois latérales desquelles sont engagées les apophyses épineuses, lesquelles prennent appui dans le fond 25 de chaque gorge G.

En outre, de façon symétrique les éléments 14 et 16 présentent chacun une première portion 26, 28 et une deuxième portion 30, 32 situées respectivement en regard l'une de l'autre et de chaque côté des deuxièmes parties d'appui 20 et 24. Les premières portions 26 et 28, prolongent de façon légèrement encaissée le bord des deuxièmes parties d'appui 20 et 24, sensiblement parallèlement aux plans moyens P1 et P2 et s'étendent des parois antérieures 15 aux parois postérieures 17. Les deuxièmes portions 30 et 32 des éléments 16 et 14, quant à elles, prolongent l'autre bord des deuxièmes parties d'appui 24 et 20 perpendiculairement aux plans moyens P2 et P1. De plus, les deuxièmes portions 30 et 32 s'étendent également des parois antérieures 15 aux parois postérieures 17, sensiblement parallèlement à leur première portion respective 28 et 26. Ainsi, la cale présente un volume central V, défini par les deuxièmes parties 20 et 24 des deux éléments 14 et 16 situées en regard l'une de l'autre et les secondes portions 32, 30 respectives. Le volume V débouche dans les parois antérieures 15 et les parois postérieures 17 des deux éléments 14 et 16.

En outre, chacune des deuxièmes portions 30 et 32 des éléments 16 et 14 est située au regard des premières portions respectivement 26 et 28 des éléments 14 et 16. Comme on l'expliquera dans la suite de la description, dans une première position de repos, les premières portions 26 et 28 sont respectivement espacées les unes des autres d'une distance d.

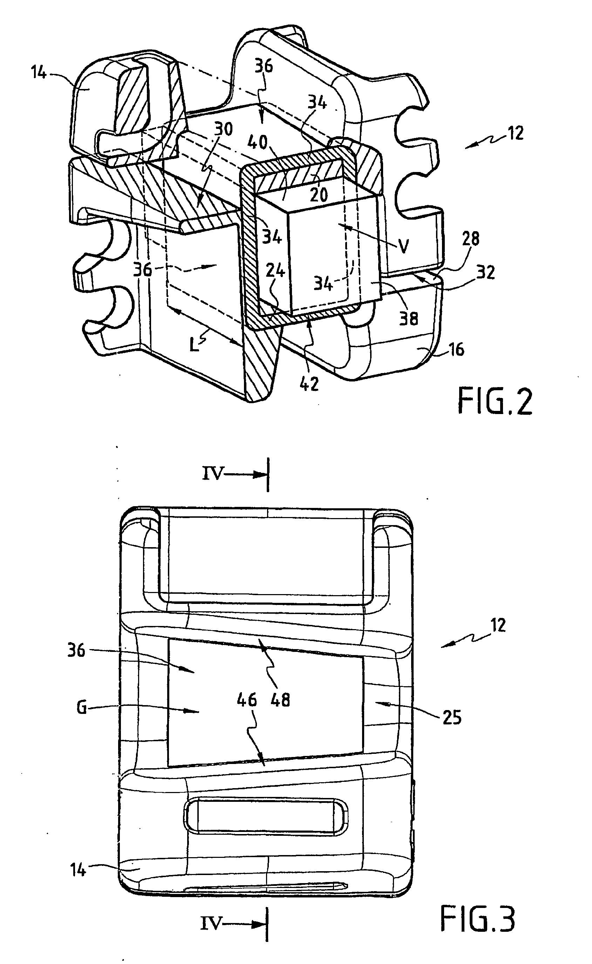

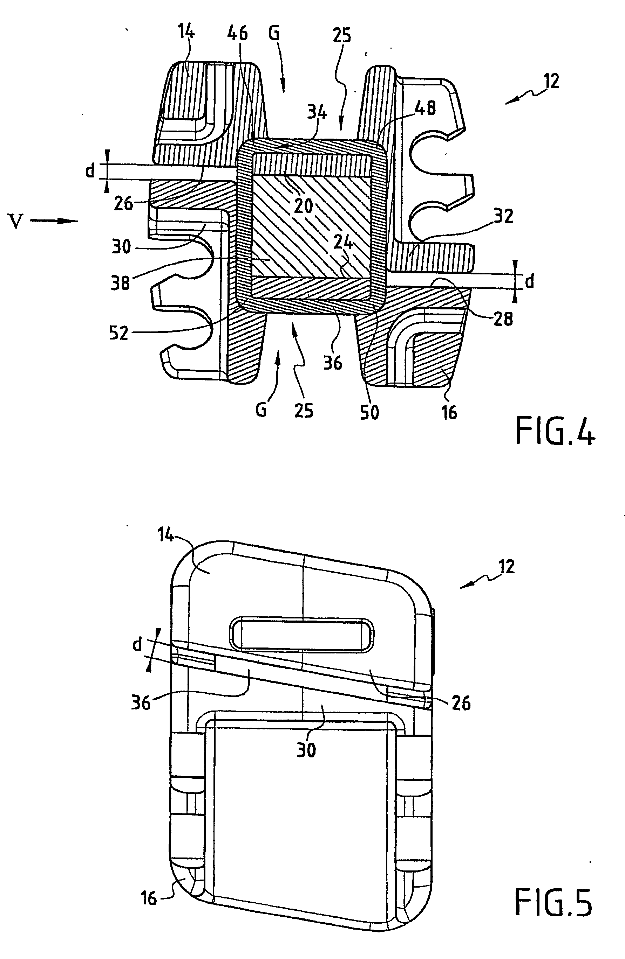

La cale 12, telle que représentée sur la Figure 1 comporte des moyens de liaison constitués d'un chemin de passage 34 traversant les deux éléments 14 et 16, et d'un lien continu 36 formant boucle empruntant ce chemin de passage 34 pour relier entre eux les éléments 14 et 16 de la cale 12. On se référera à la Figure 2 et à la Figure 4 pour décrire plus en détail les moyens de liaison des deux éléments 14 et 16 après avoir détaillé l'implant représenté en écorché sur la Figure 2 et qui comporte des moyens élastiquement compressibles 38 disposés entre les deuxièmes parties d'appui 20 et 24.

On retrouve sur la Figure 2, les deux éléments 14 et 16 dont les deuxièmes parties d'appui sont disposées en regard l'une de l'autre, et dont les deuxièmes portions 30 et 32 des éléments 16 et 14 sont respectivement disposées en regard des premières portions 26 et 28 des éléments 14 et 16.

En outre, on retrouve une portion substantielle du volume central V dont la forme est sensiblement parallélépipédique rectangle et dans lequel s'étendent entièrement les moyens élastiquement compressibles 38. Les moyens élastiquement compressibles 38 sont formés d'un seul bloc sensiblement parallélépipédique également en matériau élastomère biocompatible du type silicone par exemple. Ledit bloc présente une première paroi 40 et une deuxième paroi opposée 42 sensiblement parallèle, les deuxièmes parties d'appui 20 et 24 des éléments 14 et 16 étant en appui contre les première et deuxième parois 40 et 42 dudit bloc. Il faut souligner que la pièce élastiquement compressible 38 n'est pas liée mécaniquement, de façon positive, aux éléments 14 et 16. Il existe un simple appui entre ces pièces.

La Figure 2 illustre également le chemin de passage 34 dans lequel s'étend intégralement le lien continu 36 formant boucle, lequel est destiné à maintenir ensemble les éléments 14 et 16.

Le chemin de passage 34 décrit sensiblement un rectangle dont la largeur L est bien évidemment inférieure à la distance séparant les parois postérieure 17 et antérieure 15 de façon que les premières parties 18 et 22 des éléments 14 et 16 soient respectivement solidaires des deuxièmes parties d'appui 20 et 24.

On se référera à la Figure 4 pour décrire plus précisément le chemin de passage 34 traversant les deux éléments 14 et 16 et que le lien continu 36 formant boucle emprunte.

Le chemin de passage 34 traverse les éléments 14 et 16 respectivement dans les mêmes portions. Il apparaît dans le fond 25 de la gorge G de l'élément 14 de la cale 12 et traverse les portions de cale 46 et 48 situées à la base des parois latérales de la gorge G et débouche entre la deuxième partie d'appui 20 et la première portion 26, pour la portion de cale 46, et entre la deuxième partie d'appui 20 et la deuxième portion 32, pour la portion de cale 48. Le chemin est en outre bordé par la deuxième portion 32 jusqu'à l'élément 16 où il s'ouvre entre la deuxième partie d'appui 24 et la première portion 28 dans la paroi de cale 50 de manière analogue à la portion de cale 46 de l'élément 14. Ensuite, il traverse le fond 16 de la gorge G pour s'ouvrir dans la portion de cale 52 et s'étendre jusqu'à l'élément 14, entre la deuxième partie d'appui 20 et la deuxième portion 32, bordé par la deuxième portion 30. De la sorte, le chemin de passage forme une boucle traversant les deux éléments 14 et 16.

On comprend que le lien continu 36 qui le traverse en formant une boucle, permet de solidariser les deux éléments 14 et 16 entre eux.

La Figure 3 illustre la cale 12 en vue de dessus, où apparaît l'élément 14 et le fond 25 de la gorge G que le lien continu 36 formé d'une bande flexible, traverse. Le lien continu 36 s'engage dans les portions de cale 48 et 46 situées à la base de la gorge G.

En outre la Figure 5 illustre la cale 12 en vue de côté sur laquelle apparaissent l'élément 14 et l'élément 16 entre la première portion 26 et la deuxième portion 30 desquels est représenté le lien continu 36.

Après avoir décrit les éléments constitutifs essentiels de la cale, on décrira son mode de montage et son fonctionnement.

Selon un mode de mise en oeuvre particulièrement avantageux, les deux éléments 14 et 16 sont appliqués l'un contre l'autre, de façon que leurs premières et deuxièmes portions soient respectivement en contact. Ensuite le lien continu 36 est formé en introduisant une bande de matériau flexible dans le chemin de passage 34 et en reliant les deux extrémités de la bande par couture pour former le lien continu 36. La bande est cousue dans le fond 25 de l'une des gorges G qui est le seul endroit accessible à un dispositif de couture.

En outre, la bande est cousue de façon que le lien continu 36 soit relativement lâche lorsque les éléments 14 et 16 sont disposés l'un contre l'autre. De la sorte, dès que les éléments 14 et 16 sont entraînés dans des directions opposées l'une de l'autre le lien continu se tend et les éléments 14 et 16 sont bloqués l'un par rapport à l'autre, les premières portions 26, 28 et deuxièmes portions 30, 32 étant respectivement espacées les unes des autres d'une distance d.

Dans cette dernière position où les éléments 14 et 16 sont espacés l'un de l'autre, on introduit à force les moyens élastiquement compressibles 38 constitués d'un seul bloc. De la sorte, la cale 12 telle que représentée sur les Figures 2 et 4 se trouve dans une première position de repos, où les moyens élastiquement compressibles 38, légèrement comprimés, exercent deux forces opposées sur les deuxièmes parties d'appui 20 et 24 des éléments 14 et 16, tendant à éloigner ces derniers l'un de l'autre. Bien évidemment, les éléments 14 et 16 sont retenus par le lien continu 36.

Ainsi, on obtient une cale 12, dans laquelle des moyens élastiquement compressibles sont précontraints et dont les éléments 14 et 16 sont susceptibles d'être entraînés l'un vers l'autre avec une force déterminée, jusqu'au contact des premières 26, 28 et deuxièmes 30, 32 portions respectivement les unes contre les autres où les éléments 14 et 16 sont bloqués l'un contre l'autre.

Ladite force déterminée correspond à la compressibilité des moyens élastiquement compressibles 38. Elle correspond à la résistance que l'on souhaite imposer au rapprochement des apophyses épineuses l'une vers l'autre et elle est déterminée par le choix d'un type d'élastomère et de son état de précontrainte.

En outre, les premières parties 18 et 22 des éléments 14 et 16 étant reliées de façon rigide aux apophyses épineuses, le déplacement dans des directions opposées de ces dernières, l'une par rapport à l'autre, est limité uniquement par la mise en tension du lien continu 36. En effet, les fonds 25 de gorges G des deux éléments 14 et 16, contre lesquels le lien continu 36 est en appui, exercent alors des forces opposées l'une de l'autre produisant la tension longitudinale de la bande dudit lien continu 36.

La force avec laquelle on souhaite maintenir les apophyses épineuses l'une par rapport à l'autre en fonction des contraintes qu'elles subissent peut être déterminée par le choix d'une bande de matériau présentant un allongement sous contrainte déterminé.

Ainsi, la cale, objet de l'invention, présente l'avantage d'être élastiquement déformable en compression et d'être relativement rigide en extension puisque ces deux possibilités de déplacements relatifs sont contrôlées par deux organes distincts.

## Revendications

1. Implant intervertébral comportant une cale destinée à s'appliquer entre deux apophyses épineuses (E1, E2) de deux vertèbres (V1, V2) du rachis, **caractérisé en ce que** ladite cale comprend :
- deux éléments (14, 16) présentant chacun une première partie (18, 22) adaptée pour être reliée à une apophyse épineuse et une deuxième partie d'appui (20, 24), opposée à ladite première partie (18, 22), les deuxièmes parties d'appui (20, 24) étant situées en regard l'une de l'autre ;
- des moyens élastiquement compressibles (38) disposés entre lesdites deuxièmes parties d'appui (20, 24), lesdits moyens élastiquement compressibles (38) étant comprimés par lesdites deuxièmes parties d'appui (20, 24) lors de l'entraînement desdits deux éléments (14, 16) l'un vers l'autre ; et
- des moyens de liaison (34, 36) distincts des moyens élastiquement compressibles pour relier lesdits deux éléments (14, 16) entre eux, lesdits moyens de liaison (34, 36) permettant de bloquer lesdits deux éléments (14, 16) en translation l'un par rapport à l'autre lorsque lesdits deux éléments (14, 16) sont entraînés dans des directions opposées l'une de l'autre.

2. Implant intervertébral selon la revendication 1, **caractérisé en ce que** lesdits moyens de liaison (34, 36) comprennent au moins un chemin de passage (34) traversant chacun desdits éléments (14, 16) et débouchant sensiblement de chaque côté de ladite deuxième partie d'appui (20, 24).

3. Implant intervertébral selon la revendication 1 ou 2, **caractérisé en ce que** lesdits moyens de liaison (34, 36) comportent un lien continu (36) formant boucle, ledit lien continu (36) présentant deux premières portions opposées traversant respectivement lesdits deux éléments (14, 16) en regard.

4. Implant intervertébral selon l'une quelconque des revendications 1 à 3, **caractérisé en ce que** chacun desdits éléments (14, 16) présente au moins une première portion (26, 28) et une deuxième portion (30, 32) situées sensiblement de chaque côté de ladite deuxième partie d'appui (20, 24), la première portion (26, 28) et la deuxième portion (30, 32) de l'un desdits éléments (14, 16) étant adaptées pour s'appliquer respectivement contre la deuxième portion (30, 32) et la première portion (26, 28) de l'autre élément (16, 14) lorsque lesdits deux éléments (14, 16) sont entraînés l'un vers l'autre de façon à les bloquer en translation l'un par rapport à l'autre, les deuxièmes parties d'appui (20, 24) permettant de comprimer lesdits moyens élastiquement compressibles.

5. Implant intervertébral selon la revendication 4, **caractérisé en ce que** lesdits éléments (14, 16) présentent une paroi antérieure (15) destinée à être appliquée au regard dudit rachis et une paroi postérieure (17) opposée à ladite paroi antérieure (15) et **en ce que** ladite première portion (26, 28) et ladite deuxième portion (30, 32) desdits éléments (14, 16) s'étendent sensiblement parallèlement entre elles, de ladite paroi antérieure (15) vers ladite paroi postérieure (17).

6. Implant intervertébral selon la revendication 5, **caractérisé en ce que** lesdites deuxièmes parties d'appui (20, 24) desdits deux éléments (14, 16) situées en regard l'une de l'autre et lesdites premières portions (26, 28) et deuxièmes portions (30, 32) respectives définissent sensiblement un volume (V) débouchant dans les parois antérieures (15) et les parois postérieures (17) desdits deux éléments (14, 16), lesdits moyens élastiquement compressibles s'étendant dans ledit volume (V).

7. Implant intervertébral selon la revendication 1 et l'une quelconque des revendications 4 à 6, **caractérisé en ce que** ladite deuxième partie d'appui (20, 24) desdits éléments (14, 16) définit un plan moyen (P1, P2) et **en ce que** ladite première portion (26, 28) desdits éléments (14, 16) prolonge ladite deuxième partie d'appui (20, 24) sensiblement parallèlement audit plan moyen (P1, P2) et ladite deuxième portion (30, 32) desdits éléments (14, 16) prolonge ladite deuxième partie d'appui (26, 28) sensiblement perpendiculairement audit plan moyen (P1, P2).

8. Implant intervertébral selon les revendications 2 et 7, **caractérisé en ce que** ledit chemin de passage (34) traversant lesdits éléments (14, 16) débouche dans lesdites premières (26, 28) et deuxièmes (30, 32) portions et se prolonge sensiblement perpendiculairement auxdits plans moyens (P1, P2) desdites deuxièmes parties d'appui (20, 24).

9. Implant intervertébral selon la revendication 3, **caractérisé en ce que** ledit lien continu (36) formant boucle est constitué d'une bande continue en matériau flexible.

10. Implant intervertébral selon l'une quelconque des revendications 1 à 9, **caractérisé en ce que** lesdits éléments (14, 16) sont réalisés dans un matériau rigide.

11. Implant intervertébral selon l'une quelconque des revendications 1 à 10, **caractérisé en ce que** lesdits moyens élastiquement compressibles (38) sont formés d'une seule pièce en élastomère.

12. Implant intervertébral selon l'une quelconque des revendications 1 à 11, **caractérisé en ce que** chacune desdites premières parties (18, 22) desdits éléments (14, 16) comporte en outre des moyens d'accrochage pour relier lesdites premières parties (14, 16) auxdites apophyses épineuses (E1, E2) desdites vertèbres (V1, V2).

13. Implant intervertébral selon l'une quelconque des revendications 1 à 12, **caractérisé en ce que** les premières parties (18, 22) des éléments (14, 16) ont chacune une gorge (G) dans laquelle l'une des apophyses épineuses (E1, E2) peut s'engager.

## Patentansprüche

1. Zwischenwirbelimplantat mit einem Keil, der dafür bestimmt ist, sich zwischen zwei Dornfortsätzen (E1, E2) von zwei Wirbeln (V1, V2) der Wirbelsäule anzulegen, **dadurch gekennzeichnet, dass** der Keil folgendes umfasst:
- zwei Elemente (14, 16), die jedes einen ersten Teil (18, 22), der für die Verbindung mit einem Dornfortsatz angepasst ist, und einen zweiten Anlageteil (20, 24), der dem ersten Teil (18, 22) entgegengesetzt ist, aufweisen, wobei die zweiten Anlageteile (20, 24) einander zugewandt angeordnet sind;
- elastisch komprimierbare Mittel (38), die zwischen den zweiten Anlageteilen (20, 24) angeordnet sind, wobei die elastisch komprimierbaren Mittel (38) beim Antreiben der zwei Elemente (14, 16) zueinander hin durch die zweiten Anlageteile (20, 24) komprimiert werden; und
- Verbindungsmittel (34, 36), die von den elastisch komprimierbaren Mitteln (38) verschieden sind, für die Verbindung der zwei Elemente (14, 16) miteinander, wobei die Verbindungsmittel (34, 36) die Sperrung der zwei Elemente (14, 16) in der Verschiebung zueinander ermöglichen, wenn die zwei Elemente (14, 16) in zueinander entgegengesetzten Richtungen angetrieben werden.

2. Zwischenwirbelimplantat nach Anspruch 1, **dadurch gekennzeichnet, dass** die Verbindungsmittel (34, 36) mindestens einen Durchgangsweg (34) umfassen, der jedes der Elemente (14, 16) durchquert und im Wesentlichen beiderseits des zweiten Anlageteils (20, 24) ausmündet.

3. Zwischenwirbelimplantat nach Anspruch 1 oder 2, **dadurch gekennzeichnet, dass** die Verbindungsmittel (34, 36) eine durchgängige, eine Schleife bildende Verbindung (36) aufweisen, wobei die durchgängige Verbindung (36) zwei entgegengesetzte erste Abschnitte aufweist, die jeweils die zwei einander zugewandten Elemente (14, 16) durchqueren.

4. Zwischenwirbelimplantat nach einem der Ansprüche 1 bis 3, **dadurch gekennzeichnet, dass** jedes der Elemente (14, 16) mindestens einen ersten Abschnitt (26, 28) und einen zweiten Abschnitt (30, 32) aufweist, die im Wesentlichen beiderseits des zweiten Anlageteils (20, 24) angeordnet sind, wobei der erste Abschnitt (26, 28) und der zweite Abschnitt (30, 32) eines der Elemente (14, 16) angepasst sind, um gegen den zweiten Abschnitt (30, 32) bzw. den ersten Abschnitt (26, 28) des anderen Elements (16, 14) zur Anlage zu kommen, wenn die zwei Elemente (14, 16) zueinander hin angetrieben werden, derart, dass sie sie in der Verschiebung zueinander sperren, wobei die zweiten Anlageteile (20, 24) die Komprimierung der elastisch komprimierbaren Mittel (38) ermöglichen.

5. Zwischenwirbelimplantat nach Anspruch 4, **dadurch gekennzeichnet, dass** die Elemente (14, 16) eine vordere Wand (15) aufweisen, die für die Anlage der Wirbelsäule zugewandt bestimmt ist, und eine hintere Wand (17) aufweisen, die der vorderen Wand (15) entgegengesetzt ist, und dass der erste Abschnitt (26, 28) und der zweite Abschnitt (30, 32) der Elemente (14, 16) sich im Wesentlichen parallel zueinander erstrecken, von der vorderen Wand (15) zu der hinteren Wand (17) hin.

6. Zwischenwirbelimplantat nach Anspruch 5, **dadurch gekennzeichnet, dass** die zweiten Anlageteile (20, 24) der zwei Elemente (14, 16), die einander zugewandt angeordnet sind, und die jeweiligen ersten Abschnitte (26, 28) und zweiten Abschnitte (30, 32) im Wesentlichen einen Raum (V) definieren, der in den vorderen Wänden (15) und den hinteren Wänden (17) der zwei Elemente (14, 16) ausmündet, wobei die elastisch komprimierbaren Mittel (38) sich in den Raum (V) erstrecken.

7. Zwischenwirbelimplantat nach Anspruch 1 und einem der Ansprüche 4 bis 6, **dadurch gekennzeichnet, dass** das zweite Anlageteil (20, 24) der Elemente (14, 16) eine Mittelebene (P1, P2) definiert und dass der erste Abschnitt (26, 28) der Elemente (14, 16) den zweiten Anlageteil (20, 24) im Wesentlichen parallel zu der Mittelebene (P1, P2) fortsetzt und der zweite Abschnitt (30, 32) der Elemente (14, 16) den zweiten Anlageteil (26, 28) im Wesentlichen senkrecht zu der Mittelebene (P1, P2) fortsetzt.

8. Zwischenwirbelimplantat nach Anspruch 2 und 7, **dadurch gekennzeichnet, dass** der Durchgangsweg (34), der die Elemente (14, 16) durchquert, in den ersten (26, 28) und zweiten (30, 32) Abschnitten ausmündet und sich im Wesentlichen senkrecht zu den Mittelebenen (P1, P2) der zweiten Anlageteile (20, 24) fortsetzt.

9. Zwischenwirbelimplantat nach Anspruch 3, **dadurch gekennzeichnet, dass** die durchgängige, eine Schleife bildende Verbindung (36) von einem durchgängigen Band aus flexiblem Material gebildet ist.

10. Zwischenwirbelimplantat nach einem der Ansprüche 1 bis 9, **dadurch gekennzeichnet, dass** die Elemente (14, 16) aus einem starren Material realisiert sind.

11. Zwischenwirbelimplantat nach einem der Ansprüche 1 bis 10, **dadurch gekennzeichnet, dass** die elastisch komprimierbaren Mittel (38) aus einem einzigen Stück aus Elastomer gebildet sind.

12. Zwischenwirbelimplantat nach einem der Ansprüche 1 bis 11, **dadurch gekennzeichnet, dass** jedes der ersten Teile (18, 22) der Elemente (14, 16) ferner Einhakmittel aufweist für die Verbindung der ersten Teile (14, 16) mit den Dornfortsätzen (E1, E2) der Wirbel (V1, V2).

13. Zwischenwirbelimplantat nach einem der Ansprüche 1 bis 12, **dadurch gekennzeichnet, dass** die ersten Teile (18, 22) der Elemente (14, 16) jeder eine Rille (G) haben, mit der einer der Dornfortsätze (E1, E2) in Eingriff kommen kann.

## Claims

1. Intervertebral implant comprising a wedge which is to be applied between two spinous processes (E1, E2) of two vertebrae (V1, V2) of the spine, **characterised in that** said wedge comprises:
- two elements (14, 16) each having a first part (18, 22) adapted to be connected to a spinous process and a second support part (20, 24) opposite said first part (18, 22), the second support parts (20, 24) being situated facing one another;
- resiliently compressible means (38) arranged between said second support parts (20, 24), said resiliently compressible means (38) being compressed by said second support parts (20, 24) when said two elements (14, 16) are moved towards one another; and
- connecting means (34, 36), distinct from the resiliently compressible means, for connecting said two elements (14, 16) together, said connecting means (34, 36) allowing said two elements (14, 16) to be blocked in translation relative to one another when said two elements (14, 16) are moved in opposite directions to one another.

2. Intervertebral implant according to claim 1, **characterised in that** said connecting means (34, 36) comprise at least one passage (34) passing through each of said elements (14, 16) and opening substantially on each side of said second support part (20, 24).

3. Intervertebral implant according to either claim 1 or claim 2, **characterised in that** said connecting means (34, 36) comprise a continuous link (36) forming a loop, said continuous link (36) having two opposite first portions which pass through said two facing elements (14, 16) respectively.

4. Intervertebral implant according to any one of claims 1 to 3, **characterised in that** each of said elements (14, 16) has at least a first portion (26, 28) and a second portion (30, 32) situated substantially on each side of said second support part (20, 24), the first portion (26, 28) and the second portion (30, 32) of one of said elements (14, 16) being adapted to press against the second portion (30, 32) and the first portion (26, 28), respectively, of the other element (14, 16) when said two elements (14, 16) are moved towards one another so as to block them in translation relative to one another, the second support parts (20, 24) allowing said resiliently compressible means to be compressed.

5. Intervertebral implant according to claim 4, **characterised in that** said elements (14, 16) have an anterior wall (15) which is to be applied facing said spine and a posterior wall (17) opposite said anterior wall (15), and **in that** said first portion (26, 28) and said second portion (30, 32) of said elements (14, 16) extend substantially parallel to one another, from said anterior wall (15) to said posterior wall (17).

6. Intervertebral implant according to claim 5, **characterised in that** said second support parts (20, 24) of said two elements (14, 16) situated facing one another and said first portions (26, 28) and second portions (30, 32) define substantially a volume (V) which opens in the anterior walls (15) and the posterior walls (17) of said two elements (14, 16), said resiliently compressible means extending in said volume (V).

7. Intervertebral implant according to claim 1 and any one of claims 4 to 6, **characterised in that** said second support part (20, 24) of said elements (14, 16) defines a mid-plane (P1, P2), and **in that** said first portion (26, 28) of said elements (14, 16) extends said second support part (20, 24) substantially parallel to said mid-plane (P1, P2) and said second portion (30, 32) of said elements (14, 16) extends said second support part (26, 28) substantially perpendicularly to said mid-plane (P1, P2).

8. Intervertebral implant according to claims 2 and 7, **characterised in that** said passage (34) passing through said elements (14, 16) opens in said first (26, 28) and second (30, 32) portions and extends substantially perpendicularly to said mid-planes (P1, P2) of said second support parts (20, 24).

9. Intervertebral implant according to claim 3, **characterised in that** said continuous link (36) forming a loop is composed of a continuous strip of flexible material.

10. Intervertebral implant according to any one of claims 1 to 9, **characterised in that** said elements (14, 16) are made of a rigid material.

11. Intervertebral implant according to any one of claims 1 to 10, **characterised in that** said resiliently compressible means (38) are formed in one piece from elastomer.

12. Intervertebral implant according to any one of claims 1 to 11, **characterised in that** each of said first parts (18, 22) of said elements (14, 16) further comprises attachment means for connecting said first parts (14, 16) to said spinous processes (E1, E2) of said vertebrae (V1, V2).

13. Intervertebral implant according to any one of claims 1 to 12, **characterised in that** the first parts (18, 22) of the elements (14, 16) each have a groove (G) in which one of the spinous processes (E1, E2) is able to engage.
